# EUROPEAN PATENT APPLICATION

(11) **EP 4 545 017 A2**
(43) Date of publication of application: **30.04.2025**
(21) Application number: 24207923.4
(22) Date of filing: 21.10.2024
(51) Int. Cl.: A61B 17/12

(54) **LIGATOR**

(30) Priority: 23.10.2023 GB 202316198
(71) Applicant: Haemoband Surgical Limited, Belfast Northern Ireland BT6 8DD (GB)
(72) Inventor: GHAREEB, Essam, Enniskillen, BT94 2LS (GB); McMULLAN, Michael, Ballygawley, BT70 2ER (GB)
(74) Representative: FRKelly

(57) **Abstract**

A ligator comprises a band deployment device with annular seats spaced apart in an axial direction, each seat being configured to receive a respective ligation band. The band deployment device has an inner part and an outer part, each being configured to provide parts of the seats, the inner and outer parts being capable of relative reciprocating movement in the axial direction between a non-deploying state and a deploying state. A foremost seat is located at a free end of the deployment device from which a foremost ligation bands is deployed during use in response to operation of the deployment device from the non-deploying state to the deploying state. The foremost seat includes a ramp that is inclined upwardly in a forward direction towards the free end of the deployment device. The ramp has first and second portions with different inclinations. Roller-element bearings are provided between the inner and outer parts.

## Description

### Field of the Invention

The invention relates to ligators for applying ligating bands around tissues for the treatment of conditions such as, but not limited to, haemorrhoids.

### Background to the Invention

A ligator is a medical device for deploying ligatures. The ligature may take the form of a ligating band, and ligators for deploying ligating bands are sometimes referred to as band ligators.

Band ligation was introduced in the early 1960s and has since proven to be the most successful, non-operative method of treating haemorrhoids. It is also known to use band ligation to treat other conditions, for example oesophageal varices, whereby ligation bands are applied to dilated veins at the lower part of the oesophagus. Traditionally banding instruments required two people to perform the procedure, one person to hold a proctoscope and another to insert the banding instrument and reload the bands. Therefore, procedure times were lengthy due to the need to remove the instrument, reload the bands and reinsert it.

More recently, gun-like ligators have been developed that allow the user to deploy multiple ligating bands in sequence using a spring-biased deployment mechanism and a trigger. The spring is compressed by the initial trigger activation when deploying the first band in the sequence. The angle of the spring arms and the material used (typically stainless steel) is configured to provide relatively high force to reload bands using the reactive force of the compressed spring. A relatively high force is required so that enough force is transferred in a linear fashion to the deployment mechanism to overcome opposing frictional forces to deploy the first band, and so that there is subsequently enough force to again overcome frictional forces to reload the next band in the sequence. Such ligators tend to be incinerated after use, and are therefore typically formed from plastics. However, they need to be mechanically strong enough to support the required spring forces, and this tends to result in the ligator body having relatively thick walls, which is undesirable for environmental reasons. Furthermore, the relatively high spring force can make the ligator difficult to use.

It would be desirable to mitigate the problems outlined above.

### Summary of the Invention

From one aspect the invention provides a ligator for deploying ligation bands, the ligator comprising a band deployment device having a plurality of seats, typically annular seats, spaced apart in an axial direction, each seat being configured to receive a respective ligation band, the band deployment device comprising an inner part and an outer part each being configured to provide parts of said seats, the inner and outer parts being capable of relative reciprocating movement in said axial direction between a non-deploying state and a deploying state, wherein said seats include a foremost seat located at a free end of said deployment device from which a foremost one of said ligation bands is deployed during use in response to operation of said deployment device from the non-deploying state to the deploying state, and wherein at least said foremost seat includes a ramp that is inclined upwardly, or away from the axial direction, in a forward direction towards the free end of the deployment device. The preferred configuration is such that in response to operation of said deployment device from the non-deploying state to the deploying state, the foremost ligation band is pushed up and off the ramp of the foremost seat. Preferably the ramp of the foremost seat is provided by the inner part of the deployment device, typically by the respective seat parts of the inner part.

In preferred embodiments, the foremost seat, preferably the ramp of the foremost seat, has first and second portions, each portion having a different angle of inclination with respect to the axial direction. Preferably, the second portion is located forwardly of the first portion such that, in response to operation of the deployment device from the non-deploying state to the deploying state, the foremost band is pushed along the first portion and subsequently pushed up the second portion, and wherein the angle of inclination of the first portion is shallower than the angle of inclination of the second portion. In preferred embodiments, the first portion is inclined with respect to the axial direction at an angle of 1°-10°, preferably 5°-10°, and most preferably 7°-9°. In preferred embodiments, the second portion is inclined with respect to the axial direction at an angle of 10°-25°, preferably 15°-20°, and most preferably 16°-18°. Typically, each of said first and second portions is planar.

Advantageously, the foremost seat, typically the ramp of said foremost seat, has an upper end from which said foremost one of said ligation bands is deployed during use, said upper end being rounded.

In preferred embodiments, a plurality of recessed tracks are formed in the exterior of the inner part, the tracks being spaced apart, preferably evenly spaced apart, around the outer periphery of the inner part, each track extending in the axial direction, and wherein a respective raised portion of the inner part is defined between adjacent tracks, each raised portion extending in the axial direction, and wherein the outer part comprises a plurality of spaced apart, parallel fingers that extend in the axial direction, the inner and outer parts being configured to fit together such that each finger fits into a respective track, and each raised portion fits into a respective gap between adjacent fingers, and wherein each finger is movable along the respective track. Preferably, an outer surface of each raised portion includes a respective part of each of said seats. Preferably, an outer surface of each finger includes a respective part of at least one of the seats, optionally a respective part of all of the seats or a respective part of each seat except the foremost seat.

In preferred embodiments, one or more rolling-element bearing is provided between the inner and outer parts of the deployment device to facilitate said relative reciprocating movement, the or each rolling-element bearing preferably being a ball bearing. Preferably, at least one rolling-element bearing is provided between at least one finger, preferably each finger, and the corresponding track.

It is preferred that a plurality of rolling-element bearings are provided between each finger and the corresponding track, the rolling-element bearings being spaced apart in the axial direction. Preferably, a first rolling-element bearing is provided at a relatively forward location of the respective finger, preferably at a forward portion, for example a forward half, of the finger that includes its free end. The first rolling-element bearing is typically rearwardly spaced apart from the free end of the respective finger. Preferably, the first rolling-element bearing is located in register with, or substantially in register with, a foremost seat part formed in the respective finger, preferably being in line with, or substantially in line with, an abutment surface of the foremost seat part and/or a base of a respective ramp of the foremost seat part. Preferably, the deployment device is in the non-deploying state, the first rolling-element bearing is aligned or substantially aligned with the seat adjacent said foremost seat, preferably with an abutment surface of the seat adjacent said foremost seat part and/or a base of a respective ramp of the seat adjacent said foremost seat. It is preferred that a second rolling-element bearing is provided at a relatively rearward location of the respective finger, preferably at a rearward portion, for example a rearward half, of the finger. The second rolling-element bearing is typically located in register with, or substantially in register with, a rearmost seat part formed in the respective finger, preferably in line with, or substantially in line with, an abutment surface of the rearmost seat part and/or a base of a ramp of the rearmost seat part. Preferably, when the deployment device is in the non-deploying state, the second rolling-element bearing is aligned or substantially aligned with the rearmost seat, preferably with an abutment surface of the rearmost seat and/or a base of a respective ramp of the rearmost seat.

In preferred embodiments, the, or each, rolling-element bearing is rotatably located in a respective socket provided in an underside of the respective finger.

Preferably, an elongate recess for receiving the, or each, respective rolling-element bearing is provided in an upper face of the respective track. It is preferred that the underside of the finger is in engagement with the upper face of the respective track, with the, or each, rolling-element bearing being contained within the respective socket and the recess.

In preferred embodiments, there are five fingers.

In preferred embodiments, the arrangement is such that in response to operation of said deployment device from the non-deploying state to the deploying state, the foremost ligation band is pushed up and off the ramp of the foremost seat.

In preferred embodiments, the arrangement is such that operation of said deployment device from the non-deploying state to the deploying state involves movement of said outer part relative to said inner part in said forward direction. Said outer part is preferably configured to push the foremost ligation band up and off the ramp of the foremost seat by said movement in the forward direction. Typically, said movement in the forward direction is by a distance corresponding to a pitch between adjacent seats.

In preferred embodiments, the arrangement is such that, during operation of said deployment device from the non-deploying state to the deploying state said outer part, preferably the seat parts of the outer part, moves the or each band seated on said outer part in the forward direction to align the or each band with an adjacent forwardly located seat of the deployment device.

In preferred embodiments, the arrangement is such that operation of said deployment device from the deploying state to the non-deploying state involves movement of said outer part relative to said inner part in a rearward direction opposite to said forward direction. Preferably, said inner part, preferably the respective seat parts of said inner part, is configured to engage with the or each band carried by the deployment device when the outer part moves in said rearward direction in order to stop the or each band from moving rearwardly with the outer part and so cause the or each band to be seated in an adjacent forwardly located seat of the deployment device.

In preferred embodiments, each seat comprises a ramp that is inclined upwardly in the forward direction, wherein each seat preferably also comprising an abutment surface located rearwardly of the respective ramp.

In preferred embodiments, the ligator comprises a probe, the deployment device being provided at a free end of the probe, and an operating device typically comprising a trigger. The probe may comprise an inner tube located coaxially within an outer tube, the inner and outer tubes being capable of reciprocating movement with respect to each other in the axial direction, and wherein the inner part of the deployment device is coupled to an end of the inner tube, and the outer part of the deployment device is coupled to a corresponding end of the outer tube such that relative reciprocating movement between the inner and outer tubes causes corresponding relative reciprocating movement between the inner and outer parts. The operating device may be coupled to the probe such that operation of the operating device causes relative reciprocating movement between the inner and outer tubes and corresponding relative reciprocating movement between the inner and outer parts, the coupling between the operating device and the probe preferably being such that operation of the operating device from a first state to a second state causes the deployment device to be operated from the non-deploying state to the deploying state, and operation of the operating device from the second state to the first state causes the deployment device to be operated from the deploying state to the non-deploying state.

Typically, the deployment device includes a cavity with an open mouth at the free end of the deployment device, the ligator further including a suction system for drawing tissue, or other objects, into the cavity, and wherein the suction system may include, or be connectable to, a suction source, the deployment device typically including a through-aperture and/or channel to allow air to be drawn into the probe, in particular into the inner tube, via the mouth and cavity. Preferably, the suction system is operable to selectively create suction force at the mouth, the suction system preferably configured not to apply suction force at the mouth when the deployment device is in the non-deploying state, and to apply suction force at the mouth when the deployment device is in the deploying state.

Advantageously, the suction system is activated by the operating device, preferably such that, when the operating device is in its first state, the suction system is disabled such that suction force is not applied at the mouth, and when the operating device is in its second state the suction system is enabled such that suction force is applied at the mouth, and wherein, preferably, when the operating device is in an intermediate state between the first state and the second state, the suction system is enabled such that suction force is applied at the mouth.

Preferably, the inner tube includes an aperture through which air may be drawn when the aperture is exposed, the probe being configured such that, depending on the relative axial position of the inner and outer tubes, the aperture is either exposed or not exposed, the arrangement being such that when the deployment device is in the non-deploying state the aperture is exposed, and when the deployment device is in the deploying state the aperture is not exposed, and wherein when the suction system is activated a suction force is applied to the inner tube downstream of the aperture such that, when the aperture is exposed, the suction force draws air into the inner tube through the aperture thereby preventing the suction force from being transferred to the deployment device, and when the aperture is not exposed the suction force does not draw air into the inner tube through the aperture such that the suction force is transferred to the deployment device.

Further advantages aspects of the invention will be apparent to those ordinarily skilled in the art upon review of the following description of a specific embodiment and with reference to the accompanying drawings.

### Brief Description of the Drawings

An embodiment of the invention is now described by way of example and with reference to the accompanying drawings in which:
Figure 1 is a perspective view of a ligator embodying the invention;
Figure 1A is an enlarged perspective view of a band deployment device being part of the ligator of Figure 1;

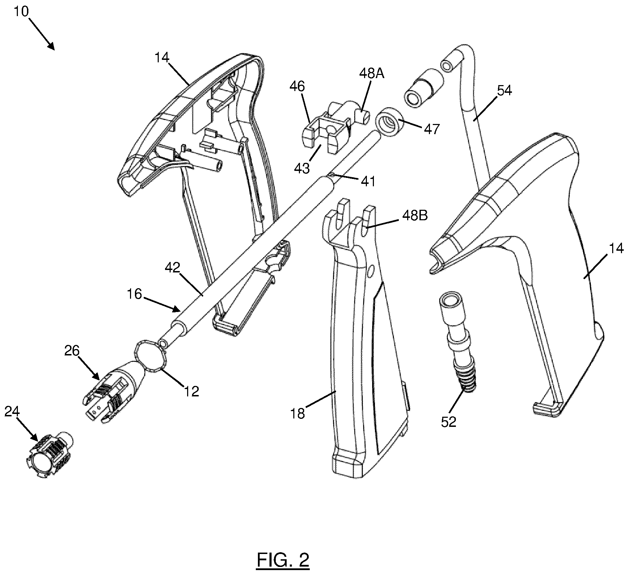

Figure 2 is an exploded perspective view of the ligator of Figure 1;
Figure 3 is a side sectioned view of the ligator of Figure 1;

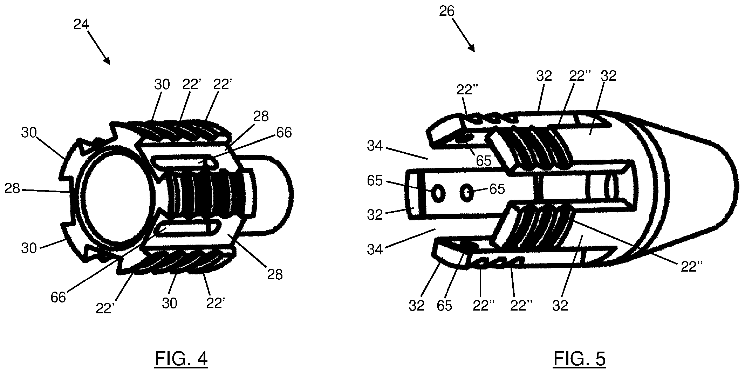

Figure 4 is a perspective view of an inner part of the band deployment device shown in Figure 1A;
Figure 5 is a perspective view of an outer part of the band deployment device shown in Figure 1A;
Figure 6 is a side sectioned view of the band deployment device shown in Figure 1A;
Figure 6A is a detail view of part of the side sectioned view of Figure 6;
Figure 7 is a side view of the band deployment device shown in a non-deploying state;
Figure 8 is a side view of the band deployment device shown in a deploying state;
Figure 9 is a side view of the band deployment device shown in the non-deploying state and loaded with four ligating bands;
Figure 10 is a side view of the band deployment device shown in the deploying state deploying a lead one of the four ligating bands; and
Figure 11 is a side view of the band deployment device returned to the non-deploying state after deployment of the leading band.

### Detailed Description of the Drawings

Referring now to the drawings there is shown, generally indicated as 10, a ligator embodying the invention. The ligator 10 is a medical device for applying ligating bands 12 around vascular structures, tissues or other formations (not shown) for the treatment of conditions such as haemorrhoids or oesophageal varices. The ligating bands 12 are elastic, typically being formed from rubber or an elastomer. The bands 12 are preferably rounded, e.g. circular in a rest state, in transverse cross-section.

The ligator 10 comprises a handle 14, preferably in the form of a hand grip, a probe 16 and an operating device typically comprising a trigger 18. The preferred ligator 10 is pistol-like in shape, such that the probe 16 may be referred to as, or likened to, a barrel. The trigger 18 may be incorporated into the handle 14 or otherwise configured for operation by a user's finger(s) when gripping the handle 14.

The probe 16 has a band deployment device 20 at its free end. The preferred deployment device 20 is configured to carry a plurality of the bands 12 simultaneously. The deployment device 20 is configured to define a respective seat 22 for each band 12. The seats 12 include a foremost seat 22A located at the tip of the probe 16, the or each other seat 22 being arranged in a row behind the foremost seat 22A. The seats 22 are spaced apart in an axial direction, i.e. along the longitudinal, or front-to-rear, axis of the band deployment device 20, which typically corresponds to the longitudinal axis of the probe 16. The seats 12 are typically annular and are arranged coaxially in a row at the end of the probe 16. In particular, each seat 22 typically comprises an annular groove that extends around the transverse periphery of the deployment device 16. In typical embodiments the deployment device 16 is circular or substantially circular in transverse cross-section and so the seats may be said to extend circumferentially around the deployment device 16. Each seat 22 is shaped and dimensioned to receive a respective band 12, the preferred arrangement being such that each band 12 is stretched, or held in tension, when seated in one of the seats 12. Preferably, the seats 12 are all of substantially the same size. In the illustrated embodiment, the ligator 10 has four seats 12 and so is capable of carrying up to four bands 12 simultaneously. In alternative embodiments, the ligator 10 may have fewer than or more than four seats, e.g. 2 seats, 3 seats or 5 seats, and more generally may be said to have at least one seat.

The deployment device 20 comprises an inner part 24 that fits into an outer part 26, the inner and outer parts 24, 26 being capable of reciprocating movement with respect to each other. In particular, when fitted together, the inner and outer parts 24, 26 are coaxial and are capable of reciprocating movement with respect to each other along a common axis (which may be said to correspond to the respective longitudinal axis of the parts 24, 26 and/or to the longitudinal, or front-to-rear, axis of the band deployment device 20). The preferred arrangement is such that the common axis is aligned or coincident with the longitudinal axis of the probe 16.

A plurality of recessed tracks 28 are formed in the exterior of the inner part 24, each track 28 running parallel with the common axis and preferably being open-ended. The tracks 28 are spaced apart, preferably evenly spaced apart, around the outer periphery of the inner part 24, i.e. circumferentially spaced apart around the inner part 24 in preferred embodiments. Between the tracks 28 are defined a plurality of raised portions 30, each raised portion 30 extending parallel with the common axis. The raised portions 30 are spaced apart, preferably evenly spaced apart, around the outer periphery of the inner part 24, i.e. circumferentially spaced apart around the inner part 24 in preferred embodiments. The outer surface of each raised portion 30 includes a respective part 22' (typically in the form of a respective groove or other recessed formation) of each of the seats 22, the respective parts of each seat 22 being in register with each other in the axial direction. As such, each raised portion 30 includes a plurality of seat parts 22', a respective one for each seat 22, arranged in a row along the common axis. Each part 22' of each seat 22 extends transversely with respect to the common axis. In the illustrated embodiment, there are five tracks 28 and five raised portions 30. In alternative embodiments there may be more or fewer tracks 28 and raised portions 30. Each of the raised portions 30 is preferably of the same, or substantially the same, size as each other (preferably in respect of all of, but optionally any one or more of, length, width and height). Each of the tracks 28 is preferably of the same, or substantially the same, size as each other (preferably in respect of all of, but optionally any one or more of, length, width and height). Optionally, the raised portions 30 and the tracks 28 are of the same, or substantially the same, size as each other in respect of length and/or width.

The outer part 26 comprises a plurality of spaced apart, parallel fingers 32, or prongs, that extend parallel with the common axis. The fingers 32 typically project in a cantilevered manner from the base of the outer part 26. In preferred embodiments, a respective finger 32 is provided for each track 28. In preferred embodiments, therefore, there are five fingers 32 although, there may be more or fewer fingers depending on the embodiment (e.g. 2-4 or 6-10 fingers). Increasing the number of fingers 32 has the effect of spreading the frictional forces required to effect reciprocating movement of the inner and outer parts 24, 26, but tends to reduce the surface area of the foremost seat 22A that can be engaged by the foremost band 12A which increases the chance that the foremost band 12A may become dislodged unintentionally, e.g. during transport. The provision of five fingers is found to provide an optimal balance between spreading the frictional forces and preventing unintentional dislodgment of the foremost band 12A.

The outer surface of each finger 32 includes a respective part 22" (typically in the form of a respective groove or other recessed formation) of at least one of the seats 22, the respective parts of the, or each, seat 22 being in register with each other in the axial direction. As such, each finger 32 may include a plurality of seat parts 22", a respective one for a respective seat 22, arranged in a row along the common axis. Each part 22" of each seat 22 extends transversely with respect to the common axis. In preferred embodiments, each finger 32 has one fewer seat part 22" than there are seats 22 (e.g. three seat parts 22" in the illustrated embodiment) although more or fewer seat parts 22" may be provided in other embodiments. The fingers 32 are spaced apart, preferably evenly spaced apart, around the outer periphery of the outer part 26, i.e. circumferentially spaced apart around the inner part 26 in preferred embodiments. Each of the fingers 32 is preferably of the same, or substantially the same, size as each other (preferably in respect of all of, but optionally any one or more of, length, width and height). Optionally, the fingers 32 and the raised portions 30 are of the same, or substantially the same, size as each other in respect of height and/or width.

The inner and outer parts 24, 26 are configured to fit together such that each finger 32 fits into a respective track 28, and each raised portion 30 fits into a respective gap 34 between adjacent fingers 32. In particular, the fingers 32 and tracks 28 are shaped and dimensioned such the fingers 32 fit into the tracks 28, preferably providing a close fit. The arrangement is such that each finger 32 is able to move back and forth along the respective track 28 in a direction parallel with the common axis. Except for the raised portions 30, the main body of the inner part 24, 26 is typically located within the space defined beneath the fingers 32.

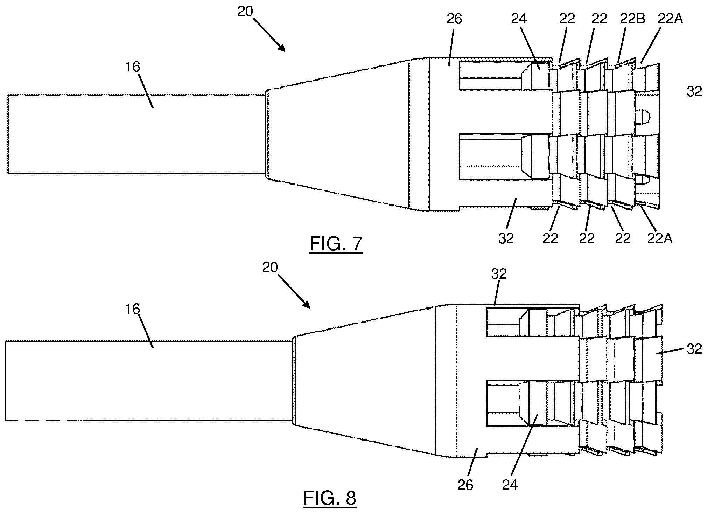

By relative reciprocating axial movement of the inner and outer parts 24, 26, the deployment device 20 is operable between a non-deploying state (shown in Fig. 7) and a deploying state (shown in Fig. 8). In the non-deploying state the inner and outer parts 24, 26 are axially further apart than when they are in the deploying state. In preferred embodiments, operation of the deployment device 20 from the non-deploying state to the deploying state is effected by moving the outer part 26 relative to the inner part 24 along their common axis in a direction towards the tip of the probe 16, and operation of the deployment device 20 from the deploying state to the non-deploying state is effected by moving the outer part 26 relative to the inner part 24 along their common axis in a direction away from the tip of the probe 16. Operation of the deployment device 20 from the non-deploying state to the deploying state causes the band 12A in the foremost seat 22A to be deployed from the ligator 10. Operation of the deployment device 20 from the deploying state to the non-deploying state results in the remaining band(s) 12 being advanced forwardly into the next seat 22 in the row.

The probe 16 comprises an inner tube 40 located coaxially within an outer tube 42, the inner and outer tubes 40, 42 being capable of reciprocating movement with respect to each other along their common axis. At least one of the inner and outer tubes 40, 42 is rigid or semi-rigid such that the probe 16 is rigid or semi-rigid. The inner part 24 of the deployment device 20 is fixed, by any convenient means, to an end of the inner tube 40. The outer part 26 of the deployment device 20 is fixed, by any convenient means, to a corresponding end of the outer tube 42.The arrangement is such that relative reciprocating movement between the inner and outer tubes 40, 42 causes corresponding relative reciprocating movement between the inner and outer parts 24, 26.

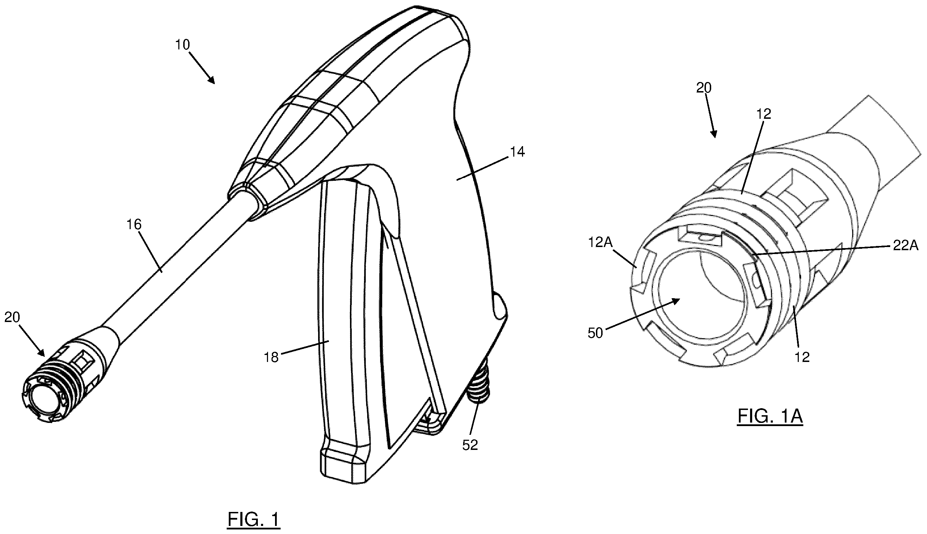

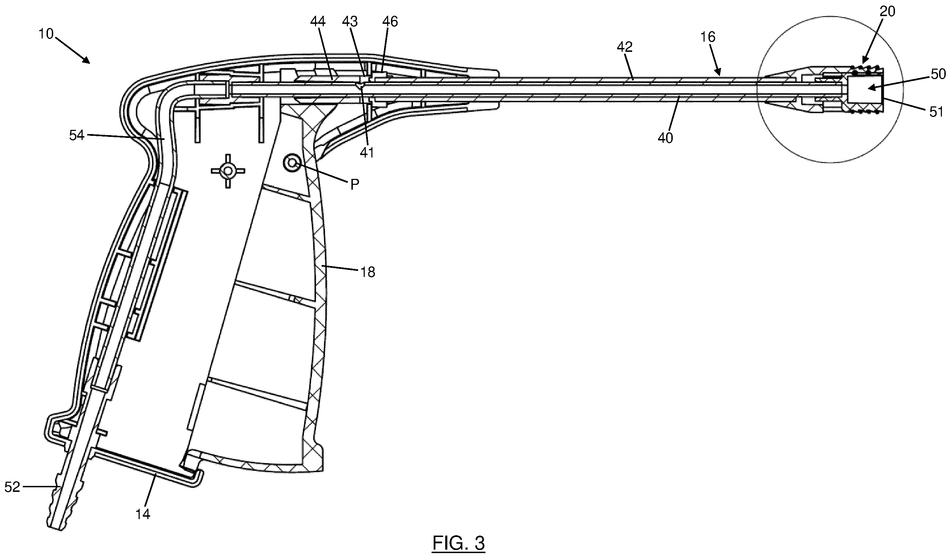

The trigger 18 is coupled to the probe 16 such that operation of the trigger 18 causes relative reciprocating movement between the inner and outer tubes 40, 42 and corresponding relative reciprocating movement between the inner and outer parts 24, 26. In preferred embodiments, the coupling between the trigger 18 and the probe 16 is such that operation of the trigger 18 from a first state (as shown in Figs 1 and 3) to a second state (not illustrated) causes the deployment device 20 to be operated from the non-deploying state to the deploying state, and operation of the trigger 18 from the second state to the first state causes the deployment device 20 to be operated from the deploying state to the non-deploying state. The trigger 18 may be pivotably coupled to the handle 14, and coupled to the probe 16, by any suitable coupling device, such that pivoting movement of the trigger 18 relative to the handle 14 causes the relative reciprocating movement between the inner and outer tubes 40, 42. In the illustrated embodiment, the trigger 18 is coupled to the handle 14 at pivot P.

In preferred embodiments, the trigger 18 is coupled to the outer tube 42 in order to move the outer tube 42 relative to the inner tube 40. The trigger 18 may be coupled to the outer tube 42 by a coupling comprising a sleeve 44 that fits around, and is slidable along, the inner tube 40 and which is connected to the outer tube 42 by a connector 46, e.g. comprising jaws. A collar 47 may be provided at the inward end of the outer tube 42 by which the connector 46 may connect with the outer tube 42. The arrangement is such that operation of the trigger 18 causes the sleeve 44 to slide along the inner tube 42 pushing or pulling the outer tube 42 such that it moves axially relative to the inner tube 40. In embodiments in which the trigger 18 is pivotable with respect to the handle 14, the sleeve 44 may be coupled to the trigger 18 by a pin 48A and slot 48B coupling assembly, or any other suitable coupling, such that pivoting movement of the trigger 18 is translated into linear movement of the outer tube 42.

It will be apparent from the foregoing that, in preferred embodiments, operation of the trigger 18 from the first state to the second state causes the outer tube 42 to move towards the tip of the probe 16 and to actuate the deployment device 20 from the non-deploying state to the deploying state, whereas operation of the trigger 18 from the second state to the first state causes the outer tube 42 to move away from the tip of the probe 16 and to actuate the deployment device 20 from the deploying state to the non-deploying state. The trigger 18 is therefore operable to cause the band 12A in the foremost seat 22A to be deployed from the ligator 10, and to cause the remaining band(s) 12 to be advanced forwardly into the next seat 22 in the row.

In preferred embodiments, the trigger 18 is resiliently biased to adopt the first state. This may be achieved in any convenient manner, for example by providing one or more spring (not shown) between the trigger 18 and the handle 14, the spring(s) being arranged to urge the trigger 18 into the first state. In preferred embodiments, a torsion spring is provided in the handle 14 and is coupled between the handle 14 and the trigger 18 to urge the trigger 18 into the first state. In use, a user may squeeze or otherwise move the trigger 18 from the first state to the second state against the bias of the spring(s), and when the user releases the trigger 18 it returns to the first state under the action of the spring(s).

In preferred embodiments, the deployment device 20 includes a cavity 50 with an open mouth 51 at the free end of the device 20, i.e. at the tip of the probe 16. Conveniently the inner part 22 of the deployment device 20 is configured to provide the cavity 50. In use, a quantity of tissue (not shown) may be drawn into the cavity 50 to facilitate deployment of a ligating band 12 onto it. In preferred embodiments, the ligator 10 includes a suction system for drawing the tissue, or other object, into the cavity 50. The deployment device 20 may therefore be said to comprise a suction nozzle. The suction system may include, or be connectable to, any conventional suction source (or a vacuum source), e.g. a suction pump or vacuum pump (not shown). In the illustrated embodiment, the suction source is not part of the ligator 10, which instead includes a connector 52 for connecting to the suction source. The deployment device 20 includes a through-aperture and/or channel 25, 27 to allow air to be drawn into the probe 16, and in particular into the inner tube 40, via the mouth 51 and cavity 50. A suction tube 54 may connect the inner tube 40 to the connector 52 or to the suction source as applicable, or the inner tube 40 may be connected directly to the connector 52 or to the suction source as applicable.

The suction system is operable to selectively create suction force at the mouth 51. In preferred embodiments, the suction system is configured not to apply suction force at the mouth 51 when the deployment device 20 is in the non-deploying mode, and to apply suction force at the mouth 51 when the deployment device 20 is in the deploying mode. In preferred embodiments, the suction system is activated by the trigger 18. The preferred arrangement is such that, when the trigger 18 is in its first state, the suction system is disabled such that suction force is not applied at the mouth 51, and when the trigger 18 is in its second state the suction system is enabled such that suction force is applied at the mouth 51. It is further preferred that, when the trigger 18 is in an intermediate state between the first state and the second state, the suction system is enabled such that suction force is applied at the mouth 51. In the intermediate state, the trigger 18 is depressed or otherwise moved out of the first state but not to the extent that the band 12A in the foremost seat 22A is deployed from the ligator 10.

In preferred embodiments, the inner tube 40 includes an aperture 41 through which air may be drawn when the aperture 41 is exposed, i.e. open to the environment. The probe 16 is configured such that, depending on the relative axial position of the inner and outer tubes 40, 42, the aperture 41 is either exposed or not exposed. In particular, the arrangement is such that when the deployment device 20 is in the non-deploying state, the aperture 41 is exposed, and when the deployment device 20 is in the deploying state, the aperture 41 is not exposed. It is further preferred that the aperture 41 is not exposed when the trigger 18 is in the intermediate state. In the illustrated embodiment the connector 46 for the outer tube 42 is configured to, e.g. shaped and dimensioned to, define a gap 43 between the sleeve 44 and the end of the outer tube 42. The arrangement is such that, when the deployment device 20 is in the non-deploying state, the aperture 41 is aligned with the gap 43 and is therefore exposed, and when the deployment device 20 is in the deploying state, or when the trigger 18 is in the intermediate state, the aperture 41 is not aligned with the gap 43, i.e. is covered by the sleeve 44 is this example, and is therefore not exposed.

When the suction system is activated (e.g. when the connector 52 is connected to the suction source and the suction source is turned on), a suction force is applied to the probe 16, in particular in the inner tube 40, downstream of the aperture 41. When the aperture 41 is exposed, the suction force draws air into the inner tube 40 through the aperture 41 thereby preventing the suction force from being transferred to the deployment device 20. When the aperture 41 is not exposed, the suction force does not draw air into the inner tube 40 through the aperture 41 and so the suction force is transferred to the deployment device 20.

In use, with the trigger 18 in its first state and no suction force being applied at the mouth 51, the tip of the probe 16 may be brought into contact with, or otherwise close to, the target tissue (or other object as applicable). The trigger 18 may then be depressed or otherwise operated to adopt the intermediate state causing suction force to be applied at the mouth 51 to draw the target tissue into the cavity 50. The trigger 18 may then be further depressed or otherwise operated to cause the foremost band 12A to be deployed onto the target tissue. When the trigger 18 is subsequently released, it adopts the first state thereby disabling the suction force at the mouth 51 and advancing the remaining band(s) 12 along the row of seats 22. For example, in the case of ligating haemorrhoids, the probe 14 may be introduced to the anal canal, typically with the assistance of a proctoscope, the mouth 51 is pressed against the targeted area of the mucosa membrane within the anal canal and the suction force is applied at the mouth by depressing the trigger 18 to the intermediate state. The targeted tissue is thus secured with the cavity 50 and the trigger 18 is then further depressed to adopt the second state causing the band 12A to be deployed to the targeted tissue. The trigger 18 is then released halting the suction which releases the banded tissue and causes the fingers 32 to retract thereby advancing the remaining bands 12, including loading the foremost remaining band 12 into the foremost seat 22A.

In preferred embodiments, the ligator 10, in particular the handle 14, probe 16, trigger 18 and deployment device 20, is formed from plastics. Preferably, the first and second parts 24, 26 of the deployment device 20 are formed from transparent material, which helps the user to correctly position the deployment device 20 during use. The spring is typically formed from metal, e.g. steel. The bands 12 may be formed from any suitable elastic material, e.g. polyisoprene or other polymer material or rubber.

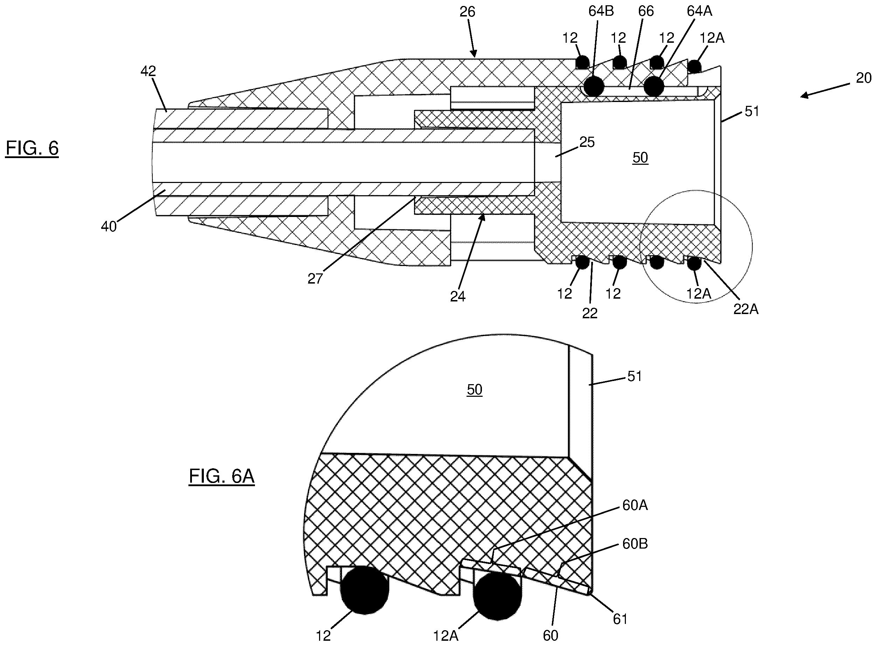

The preferred deployment device 20 and its operation are now described in more detail with reference in particular to Figures 6 to 11.

Figure 7 shows the deployment device 20 in its non-deploying state in which the outer part 26 is in a relatively retracted position with respect to the inner part 24 (i.e. relatively retracted with respect to the tip of the probe 16). The respective seat parts 22" from each row of each finger 32 are aligned, or substantially aligned, with the seat parts 22' from respective rows of the raised portions 30 of the inner part 24 to create the respective annular seat 22 that extends peripherally, or circumferentially, around the device 20. In this retracted position, the free ends of the fingers 32 are retracted with respect to the foremost seat 22A such that the foremost seat 12A is defined by the foremost seat parts 22' of the inner part 24 and not by the seat parts 22" of the fingers 32. Accordingly, each of the seats 22 except for the foremost seat 22A is formed by a combination of respective seat parts 22', 22", whereas the foremost seat 22A may be formed only by the inner part 24, although the free end of each finger 32 may provide part of the foremost seat 22A, e.g. an abutment surface as described in more detail below. As can be seen from Figure 7, in preferred embodiments the overall diameter, or width, of the inner part 24 may be smaller than the overall diameter, or width, of the outer part 26.

Figure 8 shows the deployment device 20 in its deploying state in which the outer part 26 is in a relatively advanced position with respect to the inner part 24 (i.e. relatively advanced with respect to the tip of the probe 16). In comparison with the retracted position, in the advanced position the fingers 32 are advanced with respect to the inner part 24. In particular, the outer part 26 is advanced to an extent that enables the free ends of the fingers 32 to push the foremost band 12A (not shown in Figure 8) off the free end of the inner part 24. In preferred embodiments the outer part 26 is advanced such that the free ends of the fingers 32 are at least level with, or substantially level with, but preferably project beyond, the free end of the inner part 24 in the axial direction. In preferred embodiments, the pitch between adjacent seats 22 (and between adjacent seat parts 22', 22") is constant, i.e. the same pitch between seats 22. Typically, in moving from the non-deploying state to the deploying state, the outer part 26 is advanced by a distance equal to or greater than the pitch to facilitate the free ends of the fingers 32 pushing the foremost band 12A off the free end of the inner part 24.

In preferred embodiments, each seat 22 comprises a ramp 60 that is inclined upwards, i.e. away from the axial direction (or away from the common axis or longitudinal axis) in a direction towards the free end of the deployment device 20, i.e. towards the tip of the probe 16. Each seat preferably comprises a rearward abutment surface 62, which may be disposed perpendicularly, or substantially perpendicularly, to the axial direction of the probe 16. In preferred embodiments, the ramp 60 is located forwardly of the respective abutment surface 62 (where forward is the direction towards the tip of the probe 16). By way of example, each seat 22 may have a saw tooth, or substantially saw tooth, profile in transverse cross-section. One or both of the respective seat parts 22', 22" may be shaped to provide the ramp 60 and/or abutment surface 62 as applicable. In preferred embodiments, the seat parts 22' of the inner part 24 provide the ramp 60 for the foremost seat 22A.

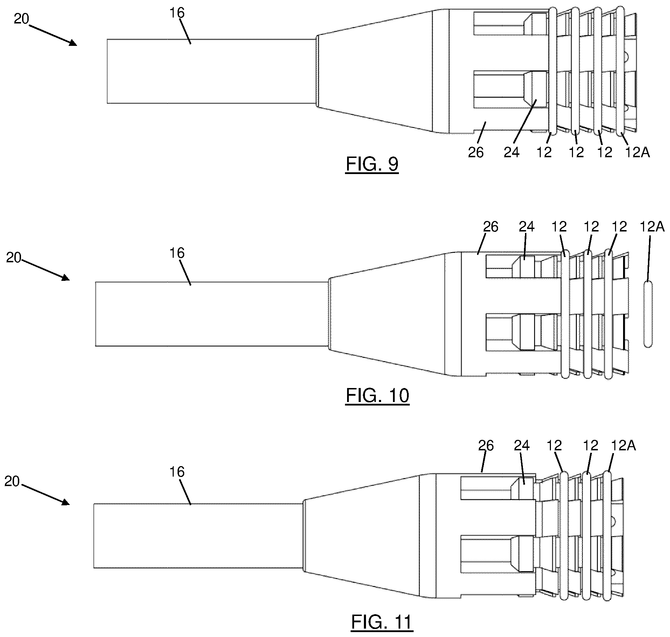

In the non-deploying state (e.g. as shown in Figures 6 and 9) a respective band 12, 12A is located in each seat 22, 22A between the abutment surface 62 and the ramp 60. Each band 12, 12A is typically located at or adjacent the bottom of the ramp 60. Each band 12, 12A is dimensioned such that it is elastically stretched and so exerts a gripping force on the respective seat 22, 22A. When the probe 16 is operated to adopt the deploying state (see for example Figure 10), forward movement of the outer part 26 causes the abutment surface 62 of the foremost seat 12A to push the foremost band 12A up the ramp 60 and off the upper end of the ramp 60, i.e. to be deployed from the probe 16 since the upper end of the ramp 60 of the foremost seat 22A corresponds to the free end of the inner part 24 and to the tip of the probe 16. In preferred embodiments, and as can be seen from Figures 8 and 10, the preferred arrangement is such that the free end of the respective fingers 32 project beyond (in the forward direction) the upper end of the ramp 60 of the foremost seat 22 in the deploying state. This helps to ensure that the foremost band 12A is deployed. The forward movement of the outer part 26 also causes the respective abutment surface 62 of each other seat 22 (in particular of the respective seat part 22") to carry the respective band 12 forwards such that it is aligned with the respective forwardly adjacent seat 22, 22A. Subsequently, as the probe 16 is returned to the non-deploying state (see for example Figure 11), rearward movement of the outer part 26 causes the remaining band(s) 12 to move along and off the upper end of the respective ramp 60 whereupon they become located in the seat 22, 22A with which they became aligned during forward movement of the outer part 26, i.e. the seat 12, 12A that is forward of the previous seat 12 in which they were located. In preferred embodiments, during the rearward movement of the outer part 26, the remaining band(s) 12 engage with the respective abutment surface 62 of the respective seat part 22' of the inner part 24, which causes the remaining band(s) 12 to move along and off the upper end of the respective ramp 60. Operation between the non-deploying state and deploying state may be repeated until each band 12 has been deployed.

During use friction forces between the inner and outer parts 24, 26 of the deployment device 20 must be overcome to effect relative reciprocating movement between the inner and outer parts 24, 26, and friction and gripping forces between the bands 12, 12A and the seats 22, 22A must be overcome to move the bands 12, 12A along the ramps 60. In particular, friction occurs between the fingers 32 and the tracks 28, and is exacerbated by the gripping force exerted by the bands 12, 12A. Bands located at or adjacent the free ends of the fingers 32 (i.e. the foremost band 12A and typically also the next foremost band 12) have a particular effect in increasing friction because of the cantilever configuration of the fingers 32 and their inherent flexibility. As a result, the force required to deploy the foremost band 12A and advance the other bands 12 can be relatively high. One way to accommodate the required forces is to make the components of the ligator 10 from suitably strong material, e.g. using relatively thick plastics or otherwise using a relatively high quantity of plastics. However, not only does this increase the cost of the ligator 10, but it is undesirable for environmental reasons particularly in cases where the ligator 10 is a single-use disposable device.

As outlined above, the force required to deploy the foremost band 12A has a particularly significant effect on the level of force required to operate the ligator 10. Preferred embodiments therefore include at least one of, and preferably all of, the following features that make it easier (i.e. requiring less force) to deploy the foremost band 12A:

### A. Provide an optimal number of fingers

In preferred embodiments the deployment device 20 has five fingers 32. The provision of five fingers 32 is found to be optimal with regard to spreading the frictional forces required to effect reciprocating movement of the inner and outer parts 24, 26 and preventing unintentional dislodgment of the foremost band 12A;

### B. Provide bearings between the inner and outer parts of the deployment device

In preferred embodiments, one or more rolling-element bearing 64 is provided between the inner and outer parts 24, 26 of the deployment device 20. In particular, at least one rolling-element bearing 64 is provided between each finger 32 and the corresponding track 28. Optionally, rolling-element bearing(s) 64 may be provided for some (typically at least two fingers, and preferably oppositely disposed fingers) but not all of the fingers 32. However, for balance it is preferred that rolling-element bearing(s) 64 are provided for all of the fingers 32. Preferably, each rolling-element bearing 64 is a ball bearing, although other types of bearing, e.g. a roller bearing could be used. In the preferred embodiment, each rolling-element bearing 64 is rotatably located in a respective socket 65 provided in the underside of the respective finger 32. An elongate recess 66, which preferably extends in the axial direction, for receiving the rolling-element bearing(s) is preferably provided in the upper face of the respective track 28. The preferred arrangement is such that, when the inner and outer parts 24 are assembled, the underside of the finger 32 is in engagement with the upper face of the respective track 28, with the rolling-element bearing(s) 64 being contained within the respective socket 65 and the recess 66. During reciprocating movement between the inner and outer parts 24, 26, the rolling-element bearing(s) 64 rotate in the respective socket 65 and run along the recess 66. In preferred embodiments, two rolling-bearing elements 64 are provided for each finger 32. In alternative embodiments, a single rolling-bearing element 64 or more than two may be provided for each finger 32. In embodiments where there are two or more rolling-bearing elements 64, they are preferably spaced-apart in the axial direction of the probe 16.

Preferably, a first rolling-element bearing 64A is provided at a relatively forward location of the respective finger 32 (i.e. at the forward portion, e.g. forward half, of the finger 32 that includes its free end). The first rolling-element bearing 64A is preferably located rearwardly of the free end of the respective finger 32. In particular, it is preferred that the first rolling-element bearing 64A is located in register with, or substantially in register with, the foremost seat part 22" formed in the respective finger 32 (preferably in line with, or substantially in line with, the respective abutment surface 62 and/or the base of the respective ramp 60). As such, when the deployment device 20 is in the non-deploying state, the first rolling-element bearing 64A is aligned or substantially aligned with the second foremost seat 22B. The preferred location of the first rolling-element bearing 64A is advantageous in that, in addition to reducing friction between the finger 32 and the track 28, the free end of the finger 32 is relatively flexible (because it is not supported by a bearing directly below it), which facilitates deployment of the foremost band 12A.

Preferably, a second rolling-element bearing 64B is provided at a relatively rearward location of the respective finger 32 (i.e. at the rearward portion, e.g. rearward half, of the finger 32 that includes its cantilevered end). It is preferred that the second rolling-element bearing 64B is located in register with, or substantially in register with, the rearmost seat part 22" formed in the respective finger 32 (preferably in line with, or substantially in line with, the respective abutment surface 62 and/or the base of the respective ramp 60). As such, when the deployment device 20 is in the non-deploying state, the second rolling-element bearing 64B is aligned or substantially aligned with the rearmost seat 22. The preferred location of the second rolling-element bearing 64B is advantageous in that, in addition to reducing friction between the finger 32 and the track 28, the second rolling-element bearing 64B restricts the amount of bending in the finger 32 during use, particularly in typical embodiments in which, in a rest state, the underside of the finger 32 does not engage with the upper surface of the corresponding track 28 such that bending of the finger may otherwise occur as a result of the gripping force exerted by the stretched band(s).

### C. Seat profile

The respective ramp 60 of each seat 22 serves to retain the respective band 12 in the seat 22 when the ligator 10 is in the non-deploying state, while allowing the band 12 to be pushed up and off the ramp 60 in response to relative reciprocating movement between the inner and outer parts 24, 26. It is found that the profile of the foremost seat 22A in particular can have a significant effect on the force required to operate the ligator 10. For example if the foremost seat 22A is not inclined, or is inclined at an angle that is too shallow, then the foremost band 12A can be displaced from the ligator 10 accidentally in the non-deploying state (e.g. during transport or at other times when the foremost band is not intended to be deployed), whereas if the profile of the foremost seat 22A, in particular its ramp 60, is inclined at an angle that is too steep, the force required to push the foremost band 12A up and off the ramp 60 during deployment can be excessive. In preferred embodiments, the foremost seat 22A, preferably the ramp 60 of the foremost seat 22A, has first and second portions 60A, 60B, each portion 60A, 60B having a different angle of inclination with respect to the axial direction of the probe 16. The first portion 60A is located rearwardly of the second portion 60B such that, during deployment, the band 12A is first pushed along the first portion 60A and is then pushed up the second portion 60B. The inclination of the first portion 60A is shallower, i.e. less steep, than the inclination of the second portion 60A. For example, the first portion 60A may be inclined with respect to the axial direction at an angle of 1 °-10°, preferably 5°-10°, and most preferably 7°-9°. Optionally, the first portion 60A may not be inclined, e.g. having an angle of 0° with respect to the axial direction. The second portion 60B may be inclined with respect to the axial direction at an angle of 10°-25°, preferably 15°-20°, and most preferably 16°-18°. In preferred embodiments, each portion 60A, 60B is planar, although one or both portion 60A, 60B may alternatively be curved. The portions 60A may be of equal length or substantially equal length. During deployment of the foremost band 12A, the relatively shallow first portion 60A is advantageous in that it facilitates the initial movement of band 12A during which static friction is overcome and the band 12A begins to move towards the upper end of the ramp 60. The relatively steep inclination of the second portion 60B inhibits accidental deployment of the foremost band 12 but, during intentional deployment, when the band 12A reaches the steeper second portion 60B, it is already moving and so static friction does not need to be overcome and pushing the band 12A up the steeper second portion 60B does not require excessive force.

In preferred embodiments, the angle of inclination of the first and second portions 60A, 60B of the foremost seat 22A is shallower than the angle of inclination of the ramps 60 of the other seats 22. For example, the ramps 60 of the other seats 22 may be inclined at an angle of 20°-25°, preferably 21°. The ramps 60 of the other seats 22 typically have a constant angle of inclination. In alternative embodiments (not illustrated), the ramps 60 of the other seats 22 may have a variable angle of inclination in a manner the same or similar to that of the ramp 60 of the foremost seat 22A, i.e. a relatively shallow portion followed by a relatively steep portion in the direction of band deployment. Optionally, one or more of the other seats 22 may include a non-inclined portion at the base of the ramp 60, e.g. having an angle of 0° with respect to the axial direction.

In preferred embodiments, the upper end 61, or edge, of the foremost seat 22A, in particular the ramp 60 of the foremost seat 22A, is rounded, which facilitates deployment of the foremost band 12A. For example, the upper end of the ramp 60 may be rounded with a radius of approximately 10mm. The rounded edge facilitates smooth deployment of the foremost band 12A onto tissue. In contrast a sharp edge may cause the band 12 to snap onto the tissue such that an abrasion may occur. The upper end of the other seats 22 are preferably sharp but may be rounded. Optionally a non-inclined portion may be provided at the top of the ramp 60.

Each of features A to C contributes to reducing the force that is required for band deployment and reloading by reducing friction forces that have to be overcome. This in turn reduces the force required by the ligator 10 to exert an equal and opposite force, thereby reducing the strength requirement of the ligator 10, allowing for a reduced quantity of plastic required. In addition, the lower frictional forces reduce the required strength of the spring(s), which may for example allow a plastics spring to be used rather than metal spring(s).

The invention is not limited to the embodiment(s) described herein but can be amended or modified without departing from the scope of the present invention.

## Claims

1. A ligator for deploying ligation bands, the ligator comprising a band deployment device having a plurality of seats spaced apart in an axial direction, each seat being configured to receive a respective ligation band, the band deployment device comprising an inner part and an outer part each being configured to provide parts of said seats, the inner and outer parts being capable of relative reciprocating movement in said axial direction between a non-deploying state and a deploying state, wherein said seats include a foremost seat located at a free end of said deployment device from which a foremost one of said ligation bands is deployed during use in response to operation of said deployment device from the non-deploying state to the deploying state, and wherein at least said foremost seat includes a ramp that is inclined away from said axial direction in a forward direction towards the free end of the deployment device.

2. The ligator of claim 1, wherein the ramp of the foremost seat is provided by the inner part of the deployment device, typically by the respective seat parts of the inner part.

3. The ligator of claim 1 or 2, wherein the foremost seat, preferably the ramp of the foremost seat, has first and second portions, each portion having a different angle of inclination with respect to the axial direction, and wherein, preferably, the second portion is located forwardly of the first portion such that, in response to operation of the deployment device from the non-deploying state to the deploying state, the foremost band is pushed along the first portion and subsequently pushed up the second portion, and wherein the angle of inclination of the first portion is shallower than the angle of inclination of the second portion, and wherein, preferably, the first portion is inclined with respect to the axial direction at an angle of 1 °-10°, preferably 5°-10°, and most preferably 7°-9°, and/or wherein, preferably, the second portion is inclined with respect to the axial direction at an angle of 10°-25°, preferably 15°-20°, and most preferably 16°-18°, and/or wherein, typically, each of said first and second portions is planar.

4. The ligator of any preceding claim, wherein the foremost seat, typically the ramp of said foremost seat, has an upper end from which said foremost one of said ligation bands is deployed during use, said upper end being rounded.

5. The ligator of any preceding claim, wherein a plurality of recessed tracks are formed in the exterior of the inner part, the tracks being spaced apart, preferably evenly spaced apart, around the outer periphery of the inner part, each track extending in the axial direction, and wherein a respective raised portion of the inner part is defined between adjacent tracks, each raised portion extending in the axial direction, and wherein the outer part comprises a plurality of spaced apart, parallel fingers that extend in the axial direction, the inner and outer parts being configured to fit together such that each finger fits into a respective track, and each raised portion fits into a respective gap between adjacent fingers, and wherein each finger is movable along the respective track, and wherein, preferably, an outer surface of each raised portion includes a respective part of each of said seats, and/or wherein, preferably, an outer surface of each finger includes a respective part of at least one of the seats, optionally a respective part of all of the seats or a respective part of each seat except the foremost seat, and wherein, preferably, said plurality of fingers comprises five fingers.

6. The ligator of any preceding claim, wherein one or more rolling-element bearing is provided between the inner and outer parts of the deployment device to facilitate said relative reciprocating movement, the or each rolling-element bearing preferably being a ball bearing.

7. The ligator of claim 6 when dependent on claim 5, wherein at least one rolling-element bearing is provided between at least one finger, preferably each finger, and the corresponding track, and wherein, preferably, a plurality of rolling-element bearings are provided between each finger and the corresponding track, the rolling-element bearings being spaced apart in the axial direction, and wherein, preferably, a first rolling-element bearing is provided at a relatively forward location of the respective finger, preferably at a forward portion, for example a forward half, of the finger that includes its free end, and wherein, preferably, the first rolling-element bearing is rearwardly spaced apart from the free end of the respective finger, and wherein, preferably, the first rolling-element bearing is located in register with, or substantially in register with, a foremost seat part formed in the respective finger, preferably being in line with, or substantially in line with, an abutment surface of the foremost seat part and/or a base of a respective ramp of the foremost seat part.

8. The ligator of claim 7, wherein, when the deployment device is in the non-deploying state, the first rolling-element bearing is aligned or substantially aligned with the seat adjacent said foremost seat, preferably with an abutment surface of the seat adjacent said foremost seat part and/or a base of a respective ramp of the seat adjacent said foremost seat.

9. The ligator of any one of claims 6 to 8, wherein a second rolling-element bearing is provided at a relatively rearward location of the respective finger, preferably at a rearward portion, for example a rearward half, of the finger, and wherein, preferably, the second rolling-element bearing is located in register with, or substantially in register with, a rearmost seat part formed in the respective finger, preferably in line with, or substantially in line with, an abutment surface of the rearmost seat part and/or a base of a ramp of the rearmost seat part, and wherein, preferably, when the deployment device is in the non-deploying state, the second rolling-element bearing is aligned or substantially aligned with the rearmost seat, preferably with an abutment surface of the rearmost seat and/or a base of a respective ramp of the rearmost seat.

10. The ligator of any one of claims 7 to 9, wherein the, or each, rolling-element bearing is rotatably located in a respective socket provided in an underside of the respective finger.

11. The ligator of any one of claims 7 to 10, wherein an elongate recess for receiving the, or each, respective rolling-element bearing is provided in an upper face of the respective track, and wherein, preferably, an underside of the finger is in engagement with the upper face of the respective track, with the, or each, rolling-element bearing being contained within the respective socket and the recess.

12. The ligator of any preceding claim, wherein operation of said deployment device from the non-deploying state to the deploying state involves movement of said outer part relative to said inner part in said forward direction, and wherein, preferably, said outer part is configured to push the foremost ligation band up and off the ramp of the foremost seat by said movement in the forward direction, and/or wherein, preferably, said movement in the forward direction is by a distance corresponding to a pitch between adjacent seats, and wherein, preferably the configuration is such that during operation of said deployment device from the non-deploying state to the deploying state said outer part, preferably the seat parts of the outer part, is configured to move the or each band seated on said outer part in the forward direction to align the or each band with an adjacent forwardly located seat of the deployment device.

13. The ligator of claim 12, wherein operation of said deployment device from the deploying state to the non-deploying state involves movement of said outer part relative to said inner part in a rearward direction opposite to said forward direction, and wherein, preferably, said inner part, preferably the respective seat parts of said inner part, is configured to engage with the or each band carried by the deployment device when the outer part moves in said rearward direction in order to stop the or each band from moving rearwardly with the outer part and so cause the or each band to be seated in an adjacent forwardly located seat of the deployment device.

14. The ligator of any preceding claim, further comprising a probe, the deployment device being provided at a free end of the probe, and an operating device typically comprising a trigger, and wherein, preferably, the probe comprises an inner tube located coaxially within an outer tube, the inner and outer tubes being capable of reciprocating movement with respect to each other in the axial direction, and wherein the inner part of the deployment device is coupled to an end of the inner tube, and the outer part of the deployment device is coupled to a corresponding end of the outer tube such that relative reciprocating movement between the inner and outer tubes causes corresponding relative reciprocating movement between the inner and outer parts, and wherein, preferably, the operating device is coupled to the probe such that operation of the operating device causes relative reciprocating movement between the inner and outer tubes and corresponding relative reciprocating movement between the inner and outer parts, the coupling between the operating device and the probe preferably being such that operation of the operating device from a first state to a second state causes the deployment device to be operated from the non-deploying state to the deploying state, and operation of the operating device from the second state to the first state causes the deployment device to be operated from the deploying state to the non-deploying state, and wherein, preferably, the deployment device includes a cavity with an open mouth at the free end of the deployment device, the ligator further including a suction system for drawing tissue, or other objects, into the cavity, and wherein the suction system may include, or be connectable to, a suction source, the deployment device typically including a through-aperture and/or channel to allow air to be drawn into the probe, in particular into the inner tube, via the mouth and cavity, and wherein, preferably, the suction system is operable to selectively create suction force at the mouth, the suction system preferably configured not to apply suction force at the mouth when the deployment device is in the non-deploying state, and to apply suction force at the mouth when the deployment device is in the deploying state, and wherein, preferably, the suction system is activated by the operating device, preferably such that, when the operating device is in its first state, the suction system is disabled such that suction force is not applied at the mouth, and when the operating device is in its second state the suction system is enabled such that suction force is applied at the mouth, and wherein, preferably, when the operating device is in an intermediate state between the first state and the second state, the suction system is enabled such that suction force is applied at the mouth.

15. The ligator of claim 14, wherein the inner tube includes an aperture through which air may be drawn when the aperture is exposed, the probe being configured such that, depending on the relative axial position of the inner and outer tubes, the aperture is either exposed or not exposed, the arrangement being such that when the deployment device is in the non-deploying state the aperture is exposed, and when the deployment device is in the deploying state the aperture is not exposed, and wherein when the suction system is activated a suction force is applied to the inner tube downstream of the aperture such that, when the aperture is exposed, the suction force draws air into the inner tube through the aperture thereby preventing the suction force from being transferred to the deployment device, and when the aperture is not exposed the suction force does not draw air into the inner tube through the aperture such that the suction force is transferred to the deployment device.
